# EUROPEAN PATENT SPECIFICATION

(11) **EP 4 063 523 B1**
(45) Date of publication and mention of the grant of the patent: **02.04.2025**
(21) Application number: 20939228.1
(22) Date of filing: 15.10.2020
(51) Int. Cl.: C12Q 1/70, C12Q 1/6851, C12Q 1/6848, C12N 15/11

(54) **COMPOSITION, KIT, METHOD FOR DETECTING SARS-COV-2 AND USE THEREOF**
ZUSAMMENSETZUNG, KIT, VERFAHREN ZUR DETEKTION VON SARS-COV-2 UND VERWENDUNG DAVON
COMPOSITION, KIT, PROCÉDÉ DE DÉTECTION DE SRAS-COV -2 ET UTILISATION ASSOCIÉE

(30) Priority: 03.06.2020 CN 202010496832
(43) Date of publication of application: 28.09.2022
(73) Proprietor: Sansure Biotech Inc., Changsha, Hunan 410205 (CN)
(72) Inventor: DAI, Lizhong, Changsha, Hunan 410205 (CN); DENG, Zhongping, Changsha, Hunan 410205 (CN); TAN, Deyong, Changsha, Hunan 410205 (CN); LIU, Jia, Changsha, Hunan 410205 (CN); JI, Bozhi, Changsha, Hunan 410205 (CN); YAN, Jin, Changsha, Hunan 410205 (CN); GUO, Xinwu, Changsha, Hunan 410205 (CN); REN, Xiaomei, Changsha, Hunan 410205 (CN); WU, Kang, Changsha, Hunan 410205 (CN); CHENG, Xing, Changsha, Hunan 410205 (CN)
(74) Representative: Grünecker Patent- und Rechtsanwälte PartG mbB
(86) International application number: PCT/CN2020/121026
(87) International publication number: WO 2021/243921

(56) References cited:
- CN-A- 110 982 942
- CN-A- 111 394 522
- ANONYMOUS: "Protocol: Real-time RT-PCR assays for the detection of SARS-CoV-2 Institut Pasteur", WORLD HEALTH ORGANIZATION, 24 January 2020 (2020-01-24), XP055888027, Retrieved from the Internet <URL:https://www.who.int/docs/default-source/coronaviruse/whoinhouseassays.pdf> [retrieved on 20220207]
- LI DANDAN ET AL: "Primer design for quantitative real-time PCR for the emerging Coronavirus SARS-CoV-2", THERANOSTICS, vol. 10, no. 16, 1 January 2020 (2020-01-01), AU, pages 7150 - 7162, XP055892463, ISSN: 1838-7640, Retrieved from the Internet <URL:https://www.ncbi.nlm.nih.gov/pmc/articles/PMC7330846/pdf/thnov10p7150.pdf> DOI: 10.7150/thno.47649
- MOLLAEI HAMID REZA ET AL: "Comparison five primer sets from different genome region of COVID-19 for detection of virus infection by conventional RT-PCR", IRANIAN JOURNAL OF MICROBIOLOGY, vol. 12, no. 3, 29 May 2020 (2020-05-29), IR, pages 185 - 193, XP055892466, ISSN: 2008-3289, Retrieved from the Internet <URL:https://www.ncbi.nlm.nih.gov/pmc/articles/PMC7340604/pdf/IJM-12-185.pdf> DOI: 10.18502/ijm.v12i3.3234
- BULTERYS PHILIP L ET AL: "Comparison of a laboratory-developed test targeting the envelope gene with three nucleic acid amplification tests for detection of SARS-CoV-2", JOURNAL OF CLINICAL VIROLOGY, ELSEVIER, AMSTERDAM, NL, vol. 129, 8 May 2020 (2020-05-08), XP086229120, ISSN: 1386-6532, [retrieved on 20200508], DOI: 10.1016/J.JCV.2020.104427
- WEI E. HUANG ET AL: "Comparison of a laboratory-developed test targeting the envelope gene with three nucleic acid amplification tests for detection of SARS-CoV-2", MICROBIAL BIOTECHNOLOGY, vol. 13, no. 4, 25 April 2020 (2020-04-25), GB, pages 950 - 961, XP055733462, ISSN: 1751-7915, DOI: 10.1111/1751-7915.13586
- JUNG YU JIN ET AL: "Comparative analysis of primer-probe sets for the laboratory confirmation of SARS- CoV-2", BIORXIV, 27 February 2020 (2020-02-27), XP055854850, Retrieved from the Internet <URL:http://dx.doi.org/10.1101/2020.02.25.964775> [retrieved on 20211026], DOI: 10.1101/2020.02.25.964775
- VICTOR M CORMAN ET AL.: "Detection of 2019 novel coronavirus (2019-nCoV) by real-time RT-PCR", EURO SURVEILL, vol. 25, no. 3, 23 January 2020 (2020-01-23), XP055695049, DOI: 10.2807/1560-7917.ES.2020.25.3.2000045
- WANG, XUDONG ET AL.: "Research Status and Application Discussion of 2019 Novel Coronavirus Nucleic Acid Detection", CHINESE JOURNAL OF CLINICAL LABORATORY SCIENCE, vol. 38, no. 2, February 2020 (2020-02-01), pages 81 - 84, XP055876467

## Description

### TECHNICAL FIELD

The present invention relates to the field of molecular biological detection, and in particular, to detection of SARS-CoV-2.

### BACKGROUND

Novel coronavirus (2019-nCoV, SARS-CoV-2) is named SARS-CoV-2 by the International Committee on Taxonomy of Viruses on February 11. Coronavirus is a large family of viruses, six viruses of which are previously known to infect humans, such as causing influenza, Middle East Respiratory Syndrome (MERS) and Severe Acute Respiratory Syndrome (SARS). SARS-CoV-2 is a new strain of coronavirus that has never been found in humans before. The virus is taxonomically a β-type coronavirus in the genus Coronavims of the family Coronaviridae. A genome with envelope and spike cytology features is a linear single-strand positive-stranded RNA ((+)ssRNA) virus. Since the discovery of SARS-CoV-2 at the end of 2019, in just four months, it breaks out almost all over the world, causing more than 1.4 million infections and more than 70,000 deaths in the world, which has attracted great attention from the international community.

In the current "Novel Coronavirus Pneumonia Diagnosis and Treatment Plan (Trial Version 7)", the detection of SARS-CoV-2 nucleic acid is still the only basis for confirmed diagnosis. There are two ways to clarify nucleic acid detection in the diagnosis and treatment plan: (1) real-time quantitative PCR (qPCR) detection of nucleic acid positive; and (2) viral gene sequencing, the sequencing result being highly homologous to known SARS-CoV-2.

Although the viral gene sequencing method is more accurate, it is time-consuming and expensive, and it is difficult to apply clinically on a large scale.

Due to the above-mentioned limitations of gene sequencing, most of patients with high suspicion to be excluded are tested by qPCR clinically. Compared with the gene sequencing, qPCR has the advantages of high speed, batch size, and low price, etc.

Compositions, kits and methods of detecting SARS-CoV-2 using combinations of primers and probes specifically hybridising to the N-gene and the orf1ab gene have been disclosed in the prior art (for example LI DANDAN et *al.,* 2020, MOLLAEI HAMID REZA *et al.,* 2020, BULTERYS PHILIP L et *al.,* 2020, WEI E. HUANG *et al.,* 2020, Jung Yu Jin *et al.,* 2020).

It is reported that since the large-scale use of qPCR detection reagents for clinical diagnosis, the initial nucleic acid positive detection rate is only 30%-50%. In addition, some medical institutions have reported that the results of a few cases have changed from negative to positive after five or more detections at different times. There are many reports on the Internet at home and abroad that primers and probes provided by the WHO (for example "Protocol: Real-time RT-PCR assays for the detection of SARS-CoV-2 Institut Pasteur", World Health Organization,2020) have specificity problems, detection reagents provided by the CDC of the United States have the problem of poor detection effect, and after recovery and discharge from hospital, it is again found that the SARS-CoV-2 nucleic acid detection turns positive. The occurrence of these situations has brought great troubles to clinical diagnosis and disease control.

Therefore, there is a need in the art for a more sensitive and accurate qPCR-related product for detecting SARS-CoV-2.

### SUMMARY

To solve the problems above, the inventors have conducted numerous analytical and experimental studies, and found two specific areas on an ORF1ab gene (Genbank ID: NC_045512.2 (266..21555)) in the genome of SARS-CoV-2, to design amplification primers and probes for qPCR detection, the nucleic acid positive detection rate can be effectively improved, and the present invention is completed accordingly.

The two specific areas mentioned above are respectively: 
ACAATTCACCTAATTTAGCATGGCCTCTTATTGTAACAGCTTTAAGGGCCAATTCT
GCTGTCAAATTACAGAATAATGAGCTTAGTCCTGTTGCACTACGACAGATGTCTTGTG
CTGCCGGTACTACACAAACTGCTTGCACTGATGACAATGCGTTAGCTTACTACAAC
(SEQ ID NO: 1);
 and

ACTAGAAGGTTCTGTTGCTTATGAAAGTTTACGCCCTGACACACGTTATGTGCTC
ATGGATGGCTCTATTATTCAATTTCCTAACACCTACCTTGAAGGTTCTGTTAGAGTGGT
AACAACTTTTGATTCTGAGTACTGTAGGCACGGCACTTGTGAAAGATCAGAAGCTG
(SEQ ID NO: 2).

Therefore, in a first aspect, the present invention provides a composition for detecting SARS-CoV-2, including:
a first primer pair, used for specifically amplifying an N gene of SARS-CoV-2;
a first probe, used for detecting an amplification product of the first primer pair;
a second primer pair, used for specifically amplifying an area as shown in SEQ ID NO: 1 in an ORF1ab gene;
a second probe, used for detecting an amplification product of the second primer pair;
a third primer pair, used for specifically amplifying an area as shown in SEQ ID NO: 2 in the ORF1ab gene; and
a third probe, used for detecting an amplification product of the third primer pair.

The primers and probes used in the present invention are all applied to qPCR.

By simultaneously detecting two specific areas of the ORF1ab gene and the N gene, the composition of the present invention improves the nucleic acid positive detection rate of SARS-CoV-2, makes the detection result more accurate, and thus has positive significance for epidemic prevention and control and the treatment of confirmed patients.

In preferred embodiments, the first primer pair of the present invention specifically amplifies the following areas of the N gene (Genbank ID: NC_045512.2 (28274..29533)):

ATGGTATTTCTACTACCTAGGAACTGGGCCAGAAGCTGGACTTCCCTATGGTG
CTAACAAAGACGGCATCATATGGGTTGCAACTGAGGGAGCCTTGAATACACCAA
AAGATCACATTGGCACCCGCAATCCTGCTAACAATGCTGCAATCG (SEQ ID NO: 3).

In a specific embodiment, the first primer pair and the first probe of the present invention are:
an N gene forward primer 1: 5'-GGTATTTCTACTACCTAGGAACT-3' (SEQ ID NO: 5);
an N gene negative primer 1: 5'-TGCAGCATTGTTAGCAGGATTG-3' (SEQ ID NO: 6); and
an N gene probe 1: 5'-GCCAGAAGCTGGACTTCCCTATGGTGC-3' (SEQ ID NO: 7).

In a specific embodiment, the first primer pair and the first probe of the present invention are:
an N gene forward primer 2: 5'-ACTACCTAGGAACTGGGCCAG-3' (SEQ ID NO: 43);
an N gene negative primer 2: 5'-AGCAGGATTGCGGGTGCCAAT-3' (SEQ ID NO: 44); and
an N gene probe 2: 5'-ACTTCCCTATGGTGCTAACAAAGACGGCAT-3' (SEQ ID NO: 45).

By using the N gene primers and probes of the present invention, Ct values of the N gene are significantly moved forward, it means that the lower limit of detection of the N gene is reduced, and the N gene at a lower concentration can be detected, which also helps to detect SARS-CoV-2 of lower concentration, making the detection more sensitive and accurate.

In a specific embodiment, the second primer pair and the second probe of the present invention are:
an ORF1ab forward primer 1-1: 5'-ATTCACCTAATTTAGCATGGCCTC-3' (SEQ ID NO: 8);
an ORF1ab negative primer 1-1: 5'-ACATCTGTCGTAGTGCAACAGGAC-3' (SEQ ID NO: 9); and
an ORF1ab probe 1-1: 5'-TTGTAACAGCTTTAAGGGCCAATTCTGCTGT-3' (SEQ ID NO: 10).

In a specific embodiment, the second primer pair and the second probe of the present invention are:
an ORF1ab forward primer 1-2: 5'-ATGGCCTCTTATTGTAACAGCT-3' (SEQ ID NO: 11);
an ORF1ab negative primer 1-2: 5'-AGCAGTTTGTGTAGTACCGGCAGC-3' (SEQ ID NO: 12); and
an ORF1ab probe 1-2: 5'-CCAATTCTGCTGTCAAATTACAGAATA-3' (SEQ ID NO: 13).

In a specific embodiment, the third primer pair and the third probe of the present invention are:
an ORF1ab forward primer 2-1: 5'-GAAGGTTCTGTTGCTTATGAAAGTT-3' (SEQ ID NO: 14);
an ORF1ab negative primer 2-1: 5'-TACCACTCTAACAGAACCTTC-3' (SEQ ID NO: 15); and
an ORF1ab probe 2-1: 5'-GCCCTGACACACGTTATGTGCTCATGGA-3' (SEQ ID NO: 16).

In a specific embodiment, the third primer pair and the third probe of the present invention are:
an ORF1ab forward primer 2-2: 5'-ATGAAAGTTTACGCCCTGAC-3' (SEQ ID NO: 17);
an ORF1ab negative primer 2-2: 5'-CCGTGCCTACAGTACTCAGAATC-3' (SEQ ID NO: 18); and
an ORF1ab probe 2-2: 5'-ATGTGCTCATGGATGGCTCTATTATTCAATT-3' (SEQ ID NO: 19).

The respective specific primer pair and probe sequences respectively of the first primer pair and the first probe, the second primer pair and the second probe, and the third primer pair and the third probe can be combined with each other, as long as satisfying that each composition includes the first primer pair and the first probe, the second primer pair and the second probe, and the third primer pair and the third probe.

In an exemplary embodiment, the present invention provides a first composition, including:
a first primer pair:
an N gene forward primer 1: 5'-GGTATTTCTACTACCTAGGAACT-3' (SEQ ID NO: 5);
an N gene negative primer 1: 5'-TGCAGCATTGTTAGCAGGATTG-3' (SEQ ID NO: 6); and
a first probe:
   an N gene probe 1: 5'-GCCAGAAGCTGGACTTCCCTATGGTGC-3' (SEQ ID NO: 7);
   a second primer pair:
      an ORF1ab forward primer 1-1: 5'-ATTCACCTAATTTAGCATGGCCTC-3' (SEQ ID NO: 8);
      an ORF1ab negative primer 1-1: 5'-ACATCTGTCGTAGTGCAACAGGAC-3' (SEQ ID NO: 9); and
      a second probe:
         an ORF1ab probe 1-1: 5'-TTGTAACAGCTTTAAGGGCCAATTCTGCTGT-3' (SEQ ID NO: 10); and
         a third primer pair:
            an ORF 1ab forward primer 2-1: 5'-GAAGGTTCTGTTGCTTATGAAAGTT-3' (SEQ ID NO: 14);
            an ORF1ab negative primer 2-1: 5'-TACCACTCTAACAGAACCTTC-3' (SEQ ID NO: 15); and
            a third probe:
               an ORF1ab probe 2-1: 5'-GCCCTGACACACGTTATGTGCTCATGGA-3' (SEQ ID NO: 16).

In another exemplary embodiment, the present invention provides a second composition, including:
a first primer pair:
an N gene forward primer 1: 5'-GGTATTTCTACTACCTAGGAACT-3' (SEQ ID NO: 5);
an N gene negative primer 1: 5'-TGCAGCATTGTTAGCAGGATTG-3' (SEQ ID NO: 6); and
a first probe:
   an N gene probe 1: 5'-GCCAGAAGCTGGACTTCCCTATGGTGC-3' (SEQ ID NO: 7);
   a second primer pair:
      an ORF1ab forward primer 1-2: 5'-ATGGCCTCTTATTGTAACAGCT-3' (SEQ ID NO: 11);
      an ORF1ab negative primer 1-2: 5'-AGCAGTTTGTGTAGTACCGGCAGC-3' (SEQ ID NO: 12); and
      a second probe:
         an ORF1ab probe 1-2: 5'-CCAATTCTGCTGTCAAATTACAGAATA-3' (SEQ ID NO: 13); and
         a third primer pair:
            an ORF1ab forward primer 2-2: 5'-ATGAAAGTTTACGCCCTGAC-3' (SEQ ID NO: 17);
            an ORF1ab negative primer 2-2: 5'-CCGTGCCTACAGTACTCAGAATC-3' (SEQ ID NO: 18); and
            a third probe:
               an ORF1ab probe 2-2: 5'-ATGTGCTCATGGATGGCTCTATTATTCAATT-3' (SEQ ID NO: 19).

In yet another exemplary embodiment, the present invention provides a third composition, including:
a first primer pair:
an N gene forward primer 1: 5'-GGTATTTCTACTACCTAGGAACT-3' (SEQ ID NO: 5);
an N gene negative primer 1: 5'-TGCAGCATTGTTAGCAGGATTG-3' (SEQ ID NO: 6); and
a first probe:
   an N gene probe 1: 5'-GCCAGAAGCTGGACTTCCCTATGGTGC-3' (SEQ ID NO: 7);
   a second primer pair:
      an ORF1ab forward primer 1-1: 5'-ATTCACCTAATTTAGCATGGCCTC-3' (SEQ ID NO: 8);
      an ORF1ab negative primer 1-1: 5'-ACATCTGTCGTAGTGCAACAGGAC-3' (SEQ ID NO: 9); and
      a second probe:
         an ORF1ab probe 1-1: 5'-TTGTAACAGCTTTAAGGGCCAATTCTGCTGT-3' (SEQ ID NO: 10); and
         a third primer pair:
            an ORF1ab forward primer 2-2: 5'-ATGAAAGTTTACGCCCTGAC-3' (SEQ ID NO: 17);
            an ORF1ab negative primer 2-2: 5'-CCGTGCCTACAGTACTCAGAATC-3' (SEQ ID NO: 18); and
            a third probe:
               an ORF1ab probe 2-2: 5'-ATGTGCTCATGGATGGCTCTATTATTCAATT-3' (SEQ ID NO: 19).

In still another exemplary embodiment, the present invention provides a fourth composition, including:
a first primer pair:
an N gene forward primer 1: 5'-GGTATTTCTACTACCTAGGAACT-3' (SEQ ID NO: 5);
an N gene negative primer 1: 5'-TGCAGCATTGTTAGCAGGATTG-3' (SEQ ID NO: 6); and
a first probe:
   an N gene probe 1: 5'-GCCAGAAGCTGGACTTCCCTATGGTGC-3' (SEQ ID NO: 7);
   a second primer pair:
      an ORF1ab forward primer 1-2: 5'-ATGGCCTCTTATTGTAACAGCT-3' (SEQ ID NO: 11);
      an ORF1ab negative primer 1-2: 5'-AGCAGTTTGTGTAGTACCGGCAGC-3' (SEQ ID NO: 12); and
      a second probe:
         an ORF1ab probe 1-2: 5'-CCAATTCTGCTGTCAAATTACAGAATA-3' (SEQ ID NO: 13); and
         a third primer pair:
            an ORF1ab forward primer 2-1: 5'-GAAGGTTCTGTTGCTTATGAAAGTT-3' (SEQ ID NO: 14);
            an ORF1ab negative primer 2-1: 5'-TACCACTCTAACAGAACCTTC-3' (SEQ ID NO: 15); and
            a third probe:
               an ORF1ab probe 2-1: 5'-GCCCTGACACACGTTATGTGCTCATGGA-3' (SEQ ID NO: 16).

By using the composition as shown above, the present invention makes the positive rate of nucleic acid detection of SARS-CoV-2 higher, and moreover can detect SARS-CoV-2 with higher sensitivity, which has a positive significance for the epidemic prevention and control and the treatment of the confirmed patients.

In a specific embodiment, the composition of the present invention may further include primer pairs that can further perform nested PCR.

Furthermore, in the composition, the fluorescent reporter groups of the second probe and the third probe can be the same or different.

Furthermore, in the composition, the first probe, the second probe and the third probe carry fluorescent reporter groups that do not interfere with each other.

Herein, "not interfere with each other" means that the fluorescent reporter groups used by the probes in the composition are different, and may not affect each other's detection, that is, different channels can be used for detection. For example, FAM, HEX, ROX and CY5 can be used. These groups do not have close absorbance values and can select different channels, and thus may not interfere with each other.

In the present invention, the fluorescent reporter group can be selected from a group consisting of FAM, HEX, ROX, VIC, CY5, 5-TAMRA, TET, CY3, and JOE, and is not limited thereto.

In a specific embodiment, the fluorescent reporter group of the first probe is ROX, and the fluorescent reporter groups of the second and third probes are FAM.

Furthermore, the 3' end of the probe also has a quenching group such as BHQ1 or BHQ2.

Furthermore, the composition includes an internal standard upstream primer, an internal standard downstream primer and an internal standard probe for monitoring.

The internal standard gene is an RNaseP gene (Genbank ID: U77665.1). Preferably, the internal standard upstream primer and the internal standard downstream primer specifically amplify the following sequence:

GAATTCGGCACGAGGTGGGACTTCAGCATGGCGGTGTTTGCAGATTTGGACC
TGCGAGCGGGTTCTGACCTGAAGGCTCTGCGCGGACTTGTGGAGACAGCCGCTC
ACCTTGGCTATTCAGTTGTTGCTATCAATCATATCGTTGACTTTAAGGAAAAGAAA
CAGGAAATTGAAAAACCAGTAGCTGTTTCTGAACTCTTCACAACTTTGCCAATTG
TACAGGGAAAATCAAGACCAATTAAAATTTTAACTAGATTAACAATTATTGTC

(SEQ ID NO: 4).

In a specific embodiment, the composition includes:
an internal standard primer pair, used for specifically amplifying an area of an RNaseP gene as shown in SEQ ID NO: 4; and
an internal standard probe, used for detecting an amplification product of the internal standard primer pair.

In a specific embodiment, the composition further includes:
an internal standard upstream primer: 5'-GAGGTGGGACTTCAGCATGGC-3' (SEQ ID NO: 20);
an internal standard downstream primer: 5'-TGTTTCTTTTCCTTAAAGTCAAC-3' (SEQ ID NO: 21); and
an internal standard probe: 5'-AGATTTGGACCTGCGAGCGGGTTCTGA-3' (SEQ ID NO: 22).

Furthermore, the first, second and third probes and the internal standard probe carry fluorescent reporter groups that do not interfere with each other.

Furthermore, the fluorescent reporter group of the internal standard probe is HEX.

Furthermore, the dosage of the primer in the composition is 25-200 nM, and the dosage of the probe in the composition is 10-80 nM.

In some embodiments, the compositions of the present invention are present in the same package.

In other embodiments, the first primer pair and the first probe, the second primer pair and the second probe, and the third primer pair and the third probe of the present invention are present in separate packages, respectively.

In yet other embodiments, the first primer pair and the first probe of the present invention are present in one package, and the second primer pair and the second probe, and the third primer pair and the third probe are present in another package.

In still other embodiments, the first primer pair and the first probe, the second primer pair and the second probe, and the third primer pair and the third probe, and the internal standard primer pair and the internal standard probe of the present invention are present in separate packages, respectively.

In a second aspect, the present invention provides use of the composition of the present invention in preparation of a kit for detecting SARS-CoV-2.

In a third aspect, the present invention provides a kit for detecting SARS-CoV-2, including the composition of the present invention as described above.

Furthermore, the kit further includes at least one of dNTP, PCR buffer, and Mg²⁺.

Furthermore, the kit further includes at least one of a nucleic acid release reagent, a nucleic acid extraction reagent, reverse transcriptase, and DNA polymerase.

Furthermore, the kit further includes at least one of the nucleic acid release reagent, the nucleic acid extraction reagent, dNTP, the reverse transcriptase, the DNA polymerase, the PCR buffer, and Mg²⁺.

Furthermore, the concentration of the reverse transcriptase is 5-15 U/µL. For example, the reverse transcriptase can be murine leukemia reverse transcriptase (MMLV) or Tth enzyme. The concentration of the DNA polymerase is 3-15 U/µL. For example, the DNA polymerase can be Taq enzyme.

In a specific embodiment, the kit of the present invention includes an inverse/reverse transcriptase, a Taq enzyme, Mg²⁺, Mn²⁺, Rnasin, dNTP, a primer, a probe, and a PCR buffer.

The common PCR buffer is composed of buffer systems such as Tris-HCl, MgCl₂, KC1, and TritonX-100. In general, the total volume of a single PCR reaction tube is 20-200 µl.

In a specific embodiment, the kit of the present invention is compatible with a digital PCR amplification system, that is, it can be directly used for amplification on a digital PCR instrument.

In a fourth aspect, provided is a method for detecting SARS-CoV-2, including the following steps:
1) extracting or releasing nucleic acids from a to-be-tested sample;
2) using the composition of the present invention to perform fluorescent quantitative PCR on the nucleic acids obtained in step 1); and
3) obtaining and analyzing a result.

In the present invention, a sample for detection can be a throat swab, sputum, bronchoalveolar lavage fluid, blood, etc., but is not limited thereto.

Furthermore, the reaction conditions of the fluorescent quantitative PCR are:
reverse transcription in which the temperature is 50°C to 60°C, and the time is 15-35 minutes, 1 cycle; cDNA pre-denaturation in which the temperature is 95°C, and the time is 1-10 minutes, 1 cycle; denaturation in which the temperature is 95°C, and the time is 10-20 seconds; and annealing in which the temperature is 60°C, and the time is 20-60 seconds, 40-50 cycles.

In a specific embodiment, provided is a method for detecting SARS-CoV-2 for non-diagnostic purposes, including the following steps:
1) extracting or releasing nucleic acids from a to-be-tested sample;
2) using the composition of the present invention or the kit of the present invention to perform fluorescent quantitative PCR on the nucleic acids obtained in step 1); and
3) obtaining and analyzing a result.

Furthermore, the reaction conditions of the fluorescent quantitative PCR are:
reverse transcription in which the temperature is 50°C to 60°C, and the time is 15-35 minutes, 1 cycle; cDNA pre-denaturation in which the temperature is 95°C, and the time is 1-10 minutes, 1 cycle; denaturation in which the temperature is 95°C, and the time is 10-20 seconds; and annealing in which the temperature is 60°C, and the time is 20-60 seconds, 40-50 cycles.

Herein, the term "non-diagnostic purposes" refers to information that is not intended to obtain whether an individual is infected with SARS-CoV-2 and has pneumonia. For example, the method can detect the presence or absence of SARS-CoV-2 in test cultures in experiments for research purposes.

### BRIEF DESCRIPTION OF THE DRAWINGS

FIG. 1 is a detection result diagram of a first composition of the composition of the present invention for detecting SARS-CoV-2;
FIG. 2 is a comparison diagram of the detection effects of the first composition of the composition of the present invention and a single-primer probe composition of ORF1ab;
FIG. 3 is a comparison diagram of the detection effects of the first composition of the composition of the present invention and another single-primer probe composition of ORFlab;
FIG. 4 is a comparison diagram of the detection effects of N gene primer probes of the composition of the present invention and primer probes of the CDC of China and the CDC of the United States;
FIG. 5 is a comparison diagram of the detection effects of N gene primer probes of the composition of the present invention and primer probes of the University of Hong Kong;
FIG. 6 is a comparison diagram of the detection effects of the composition of the present invention of an ORF1ab gene and a comparative example composition (a fifth composition);
FIG. 7 is a comparison diagram of the detection effects of the composition of the present invention of the ORF1ab gene and another comparative example composition (a sixth composition);
FIG. 8 is a detection result diagram of a FAM channel of the composition of the present invention (a first composition, a third composition, or a fourth composition) for detecting SARS-CoV-2;
FIG. 9 is a detection result diagram of an ROX channel of a seventh composition of the present invention for detecting SARS-CoV-2;
FIG. 10 is a detection result diagram of an ROX channel of an eighth composition of the present invention for detecting SARS-CoV-2;
FIG. 11 is a detection result diagram of primer and probe systems designed for three specific areas of the ORF1ab gene; and
FIG. 12 is a detection result diagram of primer and probe systems designed for two specific areas of the N gene.

### DETAILED DESCRIPTION

In the present invention, the expressions "first", "second", "third" and "fourth" etc. are only used for descriptive purposes, to distinguish the defined substances, and not to define the order or priority in any way.

For a target area provided by the present invention (e.g., an area as shown in SEQ ID NO: 1, an area as shown in SEQ ID NO: 2, an area as shown in SEQ ID NO: 3, and/or an area as shown in SEQ ID NO: 4), specific amplification primers and corresponding detection probes can be designed. For example, it can be done in the following manner.

For the fluorescent quantitative PCR primers, the following principles are usually followed: 1. the primers should be designed in an area and have specificity; 2. the length of the amplification product is 80-150 bp, and the longest one should not exceed 300 bp; 3. the product cannot form a secondary structure (the free energy is less than 58.61 KJ/mol); 4. The primer length is generally between 17 bases and 25 bases, the upstream primer and the downstream primer should not differ too much; 5. the primer itself cannot have four consecutive bases of complementarity to avoid the formation of a hairpin structure; 6. the primers cannot have four consecutive bases of complementarity to avoid the formation of primer dimers; 7. the G+C content of the primers is between 40% and 60%, and 45% to 55% is the best, and the bases in the primers are randomly distributed, and evenly distributed to the greatest extent; and 8. the Tm value of the primers is between 58 degrees and 62 degrees.

According to the principles above, for the target area provided by the present invention, for example, the primers, including, but not limited to, the primers as shown in SEQ ID NOs: 5, 6, 8, 9, 11, 12, 14, 15, 17, 18, 20, 21, 43, and 44 can be designed.

For the fluorescent quantitative PCR probes, the following principles are usually followed: 1. the probes are designed, and then the primers are designed to be close to the probes as far as possible; 2. the Tm value of the probes should be between 68 and 70, and in the case of visual probes, GC-rich areas are examined carefully; 3. a chain with more C than G is selected as the probe, and the content of G more than C may reduce the reaction efficiency; in this case, another paired chain should be selected as the probe; 4. the probe should be as short as possible, generally not more than 30 bp; and 5. the DNA folding and secondary structure of the probe is detected.

According to the principles above, for the target area provided by the present invention, for example, the primers, including, but not limited to, the primers as shown in SEQ ID NOs: 7, 10, 13, 16, 19, 22, and 45 can be designed.

Common primer and probe design software, such as Primer Express, Primer Premier, Oligo, and Omiga can be used, to assist in design. According to the specific amplification area provided by the present invention, those skilled in the art can use the software to assist in the design of probes and primers under the guidance of the above-mentioned principles.

Hereinafter, the present invention is described in detail with reference to specific embodiments and examples, and the advantages and various effects of the present invention may be more clearly presented therefrom. It should be understood by those skilled in the art that these specific embodiments and examples are intended to illustrate the present invention, instead of limiting the present invention.

### Example 1. Primers and probes used in the present invention

The primers and probes used in the present invention are as shown below:
a first composition:
a first primer pair: an N gene forward primer 1 (SEQ ID NO: 5) and an N gene negative primer 1 (SEQ ID NO: 6);
a first probe: an N gene probe 1 (SEQ ID NO: 7);
a second primer pair: an ORF1ab forward primer 1-1 (SEQ ID NO: 8) and an ORF1ab negative primer 1-1 (SEQ ID NO: 9);
a second probe: an ORF1ab probe 1-1 (SEQ ID NO: 10); and
a third primer pair: an ORF1ab forward primer 2-1 (SEQ ID NO: 14) and an ORF1ab negative primer 2-1 (SEQ ID NO: 15);
a third probe: an ORF1ab probe 2-1 (SEQ ID NO: 16).
a third composition:
   a first primer pair: an N gene forward primer 1 (SEQ ID NO: 5) and an N gene negative primer 1 (SEQ ID NO: 6);
   a first probe: an N gene probe 1 (SEQ ID NO: 7);
   a second primer pair: an ORF1ab forward primer 1-1 (SEQ ID NO: 8) and an ORF1ab negative primer 1-1 (SEQ ID NO: 9);
   a second probe: an ORF1ab probe 1-1 (SEQ ID NO: 10); and
   a third primer pair: an ORF1ab forward primer 2-2 (SEQ ID NO: 17) and an ORF1ab negative primer 2-2 (SEQ ID NO: 18);
   a third probe: an ORF1ab probe 2-2 (SEQ ID NO: 19).
   a fourth composition:
      a first primer pair: an N gene forward primer 1 (SEQ ID NO: 5) and an N gene negative primer 1 (SEQ ID NO: 6);
      a first probe: an N gene probe 1 (SEQ ID NO: 7);
      a second primer pair: an ORF1ab forward primer 1-2 (SEQ ID NO: 11) and an ORF1ab negative primer 1-2 (SEQ ID NO: 12);
      a second probe: an ORF1ab probe 1-2 (SEQ ID NO: 13); and
      a third primer pair: an ORF1ab forward primer 2-1 (SEQ ID NO: 14) and an ORF1ab negative primer 2-1 (SEQ ID NO: 15);
      a third probe: an ORF1ab probe 2-1 (SEQ ID NO: 16).

Each composition further has an internal standard upstream primer, an internal standard downstream primer, and an internal standard probe (SEQ ID NOs: 20-22).

Moreover, the fluorescent reporter group of the first probe is ROX, the fluorescent reporter groups of the second and third probes are FAM, and the fluorescent reporter group of the internal standard probe is HEX. The 3' end of the probe also has a quenching group such as BHQ1 or BHQ2.

### Example 2. Method for detecting SARS-CoV-2

A sample for detection in the present invention is a throat swab, sputum, bronchoalveolar lavage fluid, or blood. The following operations are carried out:
(1) mixing a nucleic acid release agent S1014 of Sansure Biotech with a positive control at a ratio of 1:1, and lysing at room temperature for 10 minutes;
(2) according to the proportions of 26 µL of each reaction solution and 4 µL of mixed enzyme, taking a corresponding amounts of reaction solution and mixed enzyme, mixing well, and dispensing into each reaction tube at a volume of 30 µL per reaction; adding 20 µL of the lysed to-be-tested sample to each reaction tube, then covering with a PCR tube cap, instantaneously centrifuging, and placing it in a real-time fluorescent PCR instrument; and
(3) performing reaction and detection under the following cycle conditions (a FAM channel, an ROX channel and a HEX channel are selected for fluorescence acquisition):

| qPCR procedure | | | | |
|---|---|---|---|---|
| Step | | Temperature | Time | Number of cycles |
| 1 | Reverse transcription | 60°C | 15 minutes | 1 |
| 2 | cDNA pre-denaturation | 95°C | 1 minute | 1 |
| 3 | Denaturation | 95°C | 15 seconds | 45 |
| | Annealing, extension and fluorescence acquisition | 60°C | 20 seconds | |
| 4 | Instrument cooling | 25°C | 10 seconds | 1 |

After the reaction is completed, the results are automatically saved, and the amplification curves of the detection standard and the internal standard are analyzed respectively. The Start value, End value and Threshold value of Baseline are adjusted according to the image after analysis (a user can adjust it according to the actual situation, the Start value can be set between 3 and 15, and the End value can be set between 5 and 20, the amplification curve of the negative control is adjusted to be straight or below a threshold line), Analyze is clicked to analyze, so that the parameters meet the requirements in the following "Quality Control", and then the qualitative results are recorded in a Plate window.

### (4) Quality control

SARS-CoV-2-PCR-negative control: the FAM, ROX and internal standard (HEX) channels have no Ct value or Ct>40;
SARS-CoV-2-PCR-positive control: the FAM, ROX and internal standard (HEX) channels all have Ct≤35.

The requirements above should all be met in the same experiment, otherwise, the experiment is invalid and needs to be repeated.

### Example 3. Composition of the present invention for detecting SARS-CoV-2

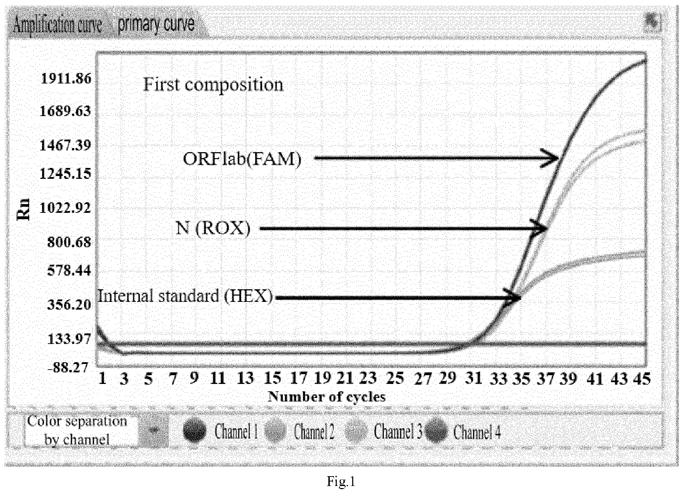

The preservation samples of novel coronavirus from the CDC of China are tested using the first composition in Example 1 of the present invention according to the method described in Example 2, and the experiment is repeated twice, and the results are as shown in FIG. 1.

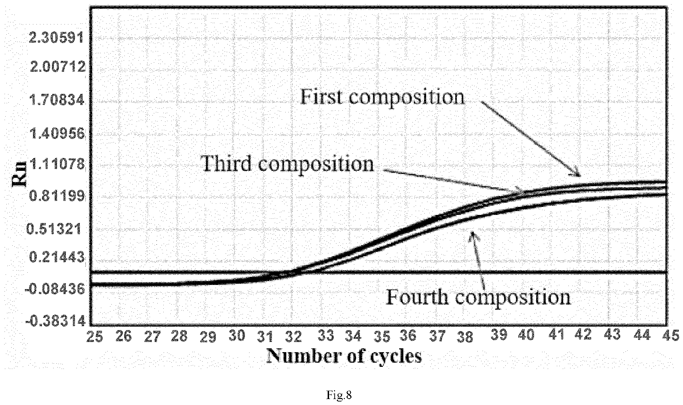

The preservation samples of the novel coronavirus from the CDC of China are tested using the first composition, the second composition, and the third composition in Example 1 of the present invention according to the method described in Example 2. FIG. 8 shows the detection result of the FAM channel.

As can be seen from FIG. 1 and FIG. 8, each amplification curve is tall and straight, and the Ct value meets the conditions, indicating that the compositions of the present invention can detect SARS-CoV-2.

### Example 4. Comparison of the detection effects of the composition of the present invention and a single-primer probe composition of ORF1ab

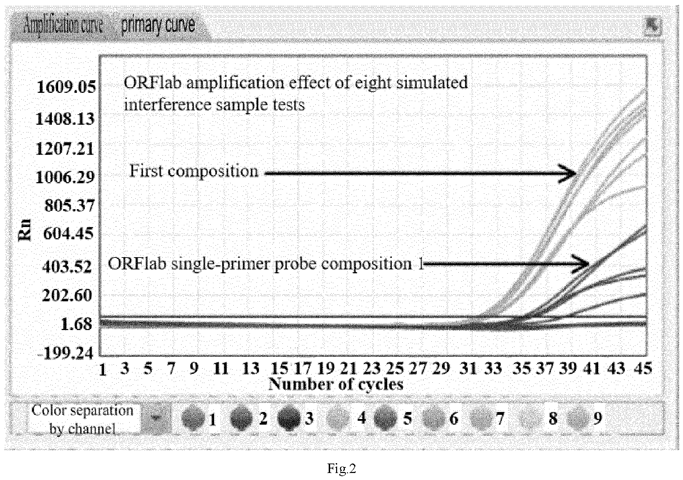

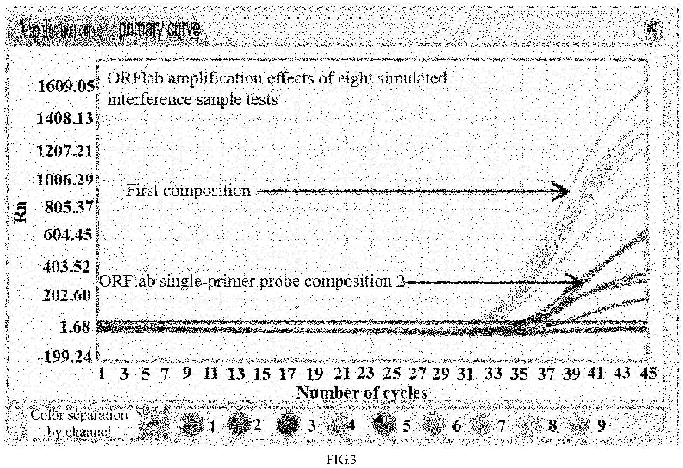

Eight cases of novel coronavirus samples with negative cotton throat swab samples stored in a preservation solution for more than one week and positive nucleic acid added as simulated interference samples are tested using the first composition described in Example 1 of the present invention, an ORF1ab single-primer probe composition 1 (excluding SEQ ID NOs: 14-16, the rest of the composition is consistent with the first composition), and an ORF1ab single-primer probe combination 2 (excluding SEQ ID NOs: 8-10, the rest of the composition is consistent with the first composition) according to the method described in Example 2. The test results are as shown in FIG. 2 and FIG. 3.

As can be seen from FIG. 2 and FIG. 3, the Ct value of the first composition of the present invention containing ORF1ab double primers is significantly moved forward than that of the single-primer probe composition, indicating that the sensitivity is higher than that of the single-primer probe composition.

### Example 5. Comparison of the detection effects of the composition of the present invention and the primer probes of the CDC of China, the CDC of the United States, and the University of Hong Kong

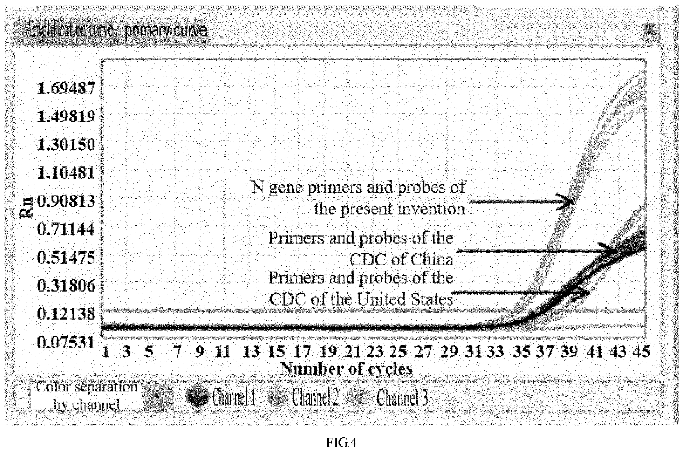

Combined detection is performed on 5-10 copies/ml of the novel coronavirus sample (a virus culture solution) by using the first primer pair and the first probe (SEQ ID NOs: 5-7) in the composition described in Example 1 of the present invention, the primer probes (three sets) of the CDC of China and the CDC of the United States (because the CDC of the United States only provides the primers and probes of the N gene, this example can only compare the difference of the N gene) according to the method described in Example 2, and the detection is repeated 10 times. The detection results are as shown in FIG. 4. As can be seen from the figure, for the novel coronavirus of the same concentration, the Ct value of the N gene detected by the first primer pair and the first probe of the present invention is significantly moved forward than that of the primer probes of the CDC of China and the CDC of the United States.

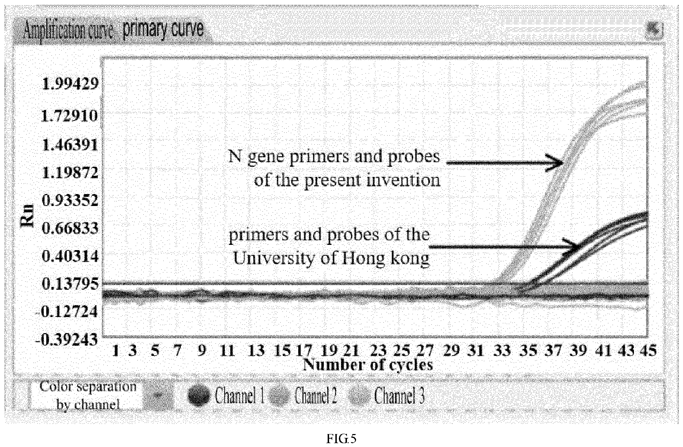

Combined detection is performed on 5-10 copies/ml of the novel coronavirus sample (the bronchoalveolar lavage fluid sample, the virus culture solution, the plasma sample) using the first primer pair and the first probe in the composition described in Example 1 of the present invention, and the primers and probes of the University of Hong Kong according to the method described in Example 2, and the detection is repeated 10 times. The detection results are as shown in FIG. 5. As can be seen from the figure, for the novel coronavirus of the same concentration, the Ct value of the N gene detected by the first primer pair and the first probe of the present invention is significantly moved forward than that of the primer probes of the University of Hong Kong.

SARS-CoV-2 can be detected more accurately by using the composition of the present invention.

The specific sequences of various primers and probes used as comparisons in this example are given below:
China CDC SARS-CoV-2 N upstream primer: 5'-GGGGAACTTCTCCTGCTAGAAT-3' (SEQ ID NO: 23);
China CDCSARS-CoV-2 N downstream primer: 5'-CAGACATTTTGCTCTCAAGCTG-3' (SEQ ID NO: 24);
China CDCSARS-CoV-2 N probe: 5'-TTGCTGCTGCTTGACAGATT-3' (SEQ ID NO: 25);
US CDCSARS-CoV-2_N1 upstream primer: 5'-GACCCCAAAATCAGCGAAAT-3' (SEQ ID NO: 26);
US CDCSARS-CoV-2_N1 downstream primer:
   5'-TCTGGTTACTGCCAGTTGAATCTG-3' (SEQ ID NO: 27);
   US CDCSARS-CoV-2_N1 probe: 5'-ACCCCGCATTACGTTTGGTGGACC-3' (SEQ ID NO: 28);
   US CDCSARS-CoV-2_N2 upstream primer: 5'-TTACAAACATTGGCCGCAAA-3' (SEQ ID NO: 29);
   US CDCSARS-CoV-2_N2 downstream primer: 5'-GCGCGACATTCCGAAGAA-3' (SEQ ID NO: 30);
   US CDCSARS-CoV-2_N2 probe: 5'-ACAATTTGCCCCCAGCGCTTCAG-3' (SEQ ID NO: 31);
   US CDCSARS-CoV-2_N3 upstream primer: 5'-GGGAGCCTTGAATACACCAAAA-3' (SEO ID NO: 32);
   US CDCSARS-CoV-2_N3 downstream primer: 5'-TGTAGCACGATTGCAGCATTG-3' (SEQ ID NO: 33);
   US CDCSARS-CoV-2_N3 probe: 5'-AYCACATTGGCACCCGCAATCCTG-3' (SEQ ID NO: 34);
   HKU-N upstream primer: 5'-TAATCAGACAAGGAACTGATTA-3' (SEQ ID NO: 35);
   HKU-N downstream primer: 5'-CGAAGGTGTGACTTCCATG-3' (SEQ ID NO: 36);
   HKU-N probe: 5'-FAM-GCAAATTGTGCAATTTGCGG-TAMRA-3' (SEQ ID NO: 37).

### Example 6. Anti-interference test of the composition of the present invention

The novel coronavirus samples with interfering substances are verified using the first composition (SEQ ID NOs: 5-10, 14-16, and 20-22) in Example 1 of the present invention according to the method described in Example 2.

Upon test and verification, the interfering substances do not significantly interfere with the test results of the kit. The experimental results are as shown in Table 1, and no interference occurs in the three repeated experiments.

**Table 1**

| Interfering substances | Concentration | Result (three repeated experiments) | Interfering substances | Concen tration | Result (three repeated experiments) |
|---|---|---|---|---|---|
| Purified mucin | 20 µg/mL | 0/3 | Dexamethason e | 50 µg/mL | 0/3 |
| Human whole blood | 5% (v/v) | 0/3 | Cefmenoxime hydrochloride | 50 µg/mL | 0/3 |
| Oxymetazoline hydrochloride | 100 µg/mL | 0/3 | Oseltamivir | 100 µg/mL | 0/3 |
| Chlorine mimatsu | 50 µg/mL | 0/3 | Zanamivir | 100 µg/mL | 0/3 |

| Interfering substances | Concentration | Result (three repeated experiments) | Interfering substances | Concen tration | Result (three repeated experiments) |
|---|---|---|---|---|---|
| Ribavirin | 100 µg/mL | 0/3 | Azithromycin | 100 µg/mL | 0/3 |
| Fluticasone | 200 µg/mL | 0/3 | Budesonide | 320 µg/mL | 0/3 |
| α-interferon | 300 µg/mL | 0/3 | Phenylephrine | 125 µg/mL | 0/3 |
| Mometasone | 100 µg/mL | 0/3 | Tobramycin | 100 µg/mL | 0/3 |
| Ritonavir | 100 µg/mL | 0/3 | Flunisolide | 100 µg/mL | 0/3 |
| Histamine hydrochloride | 200 µg/mL | 0/3 | Peramivir | 100 µg/mL | 0/3 |
| Lopinavir | 100 µg/mL | 0/3 | Mupirocin | 100 µg/mL | 0/3 |
| Triamcinolone acetonide | 100 µg/mL | 0/3 | Anhydrous ethanol | 20% (v/v) | 0/3 |
| Abidore | 100 µg/mL | 0/3 | Sodium chloride | 60 µg/mL | 0/3 |
| Urea | 100 µg/mL | 0/3 | Heme | 10 µg/mL | 0/3 |

### Example 7. Specificity verification of the composition of the present invention

The first composition (SEQ ID NOs: 5-10, 14-16, and 20-22) of the present invention does not cross-react with the positive samples such as coronaviruses (NL63, HKU1, 229E, OC43), SARS coronavirus, MERS coronavirus, influenza A virus, influenza B Yamagata, Victoria, influenza A H1N1, influenza A H3N2, influenza A H5N1, influenza A H7N9, respiratory syncytial virus types A and B, rhinovirus types A, B and C, adenovirus types 1, 2, 3, 4, 5, 7 and 55, parainfluenza virus types 1, 2, and 3, enterovirus types A and B, enterovirus C (EV-C95), enterovirus D (EV-D70), metapneumovirus, human metapneumovirus, cryptococcus neoformans, streptococcus pyogenes, acinetobacter baumannii, pneumocystis, klebsiella pneumoniae, streptococcus pneumoniae, haemophilus influenzae, pseudomonas aeruginosa, legionella pneumophila, bacillus pertussis, staphylococcus aureus, mycoplasma pneumoniae, streptococcus pneumoniae, klebsiella pneumoniae, chlamydia pneumoniae, Epstein-Barr virus, human cytomegalovirus, aspergillus fumigatus, candida albicans, candida glabrata, mycobacterium tuberculosis, nontuberculous mycobacterium, norovirus, rotavirus, aquatic sore virus, measles virus, mumps virus, and human genome DNA.

The test results for some specific examples are listed in Table 2 below.

**Table 2**

| Positive samples | Strain | Source/sample type | Concentration | Ct value (ORF1ab gene/N gene) |
|---|---|---|---|---|
| Human coronavirus 229E | 229E | Clinical sample | 1.0×10⁶ copies/ml | Not detected/not detected |
| Human coronavirus OC43 | OC43 | Clinical sample | 1.0×10⁶ copies/ml | Not detected/not detected |
| Human coronavirus HKU1 | HKU 1 | Clinical sample | 1.0×10⁶ copies/ml | Not detected/not detected |
| Human coronavirus NL63 | NL63 | Clinical sample | 1.0×10⁶ copies/ml | Not detected/not detected |
| SARS-coronavirus | | RNA | 1.0×10⁶ copies/ml | Not detected/not detected |
| MERS-coronavirus | | Pseudovirus | 1.0×10⁶ copies/ml | Not detected/not detected |
| Adenovirus 1 | | Clinical sample | 1.0×10⁶ copies/ml | Not detected/not detected |
| Human Metapneumovirus (hMPV) | | Clinical sample | 1.0×10⁶ copies/ml | Not detected/not detected |
| Parainfluenza vims 1-4 | | Clinical sample | 1.0×10⁶ copies/ml | Not detected/not detected |
| Influenza A | | National standard | 1.0×10⁶ TCID50/ L | Not detected/not detected |
| Influenza B | | Clinical sample | 1.0×10⁶ copies/ml | Not detected/not detected |
| Enterovirus (EV-C95) | | Clinical sample | 1.0×10⁶ copies/ml | Not detected/not detected |
| Respiratory syncytial virus | | Clinical sample | 1.0×10⁶ copies/ml | Not detected/not detected |
| Rhinovirus | | Clinical sample | 1.0×10⁶ copies/ml | Not detected/not detected |
| Chlamydia pneumonia | | Clinical sample | 1.0×10⁶ copies/ml | Not detected/not detected |
| Haemophilus influenzae | | National standard | 1.0×10⁶ CFU/ml | Not detected/not detected |
| Legionella pneumophila | | National standard | 1.0×10⁶ CFU/ml | Not detected/not detected |
| Mycobacterium tuberculosis | | Clinical sample | 1.0×10⁶ copies/ml | Not detected/not detected |
| Streptococus pneumonia | | National standard | 1.0×10⁶ CFU/ml | Not detected/not detected |
| Streptococcus pyogenes | | Clinical sample | 1.0×10⁶ copies/ml | Not detected/not detected |
| Bordetella pertussis | | Clinical sample | 1.0×10⁶ copies/ml | Not detected/not detected |
| Mycoplasma pneumoniae | | Clinical sample | 1.0×10⁶ copies/ml | Not detected/not detected |
| Pneumocystis jirovecii (PJP) | | Clinical sample | 1.0×10⁶ copies/ml | Not detected/not detected |

### Example 8. Precision verification of the composition of the present invention

In this experiment, SARS-CoV-2 positive samples are diluted to medium concentration positive and critical concentration positive, and SARS-CoV-2 negative samples are added as to-be-tested samples for intra-batch imprecision determination.

For each precision sample, one experimental batch is analyzed per day, and each precision sample is repeated five times for five consecutive days. A qualified novel coronavirus detection kit (including the first composition of the present invention (SEQ ID NOs: 5-10, 14-16 and 20-22)) is used for detection on the same SLAN 96P instrument, the positive detection rate and negative detection rate are analyzed, and the precision performance of the kit are investigated. The results are as shown in Table 3 below. The results show that the detection results of the composition of the present invention are consistent in each batch, reflecting good precision.

**Table 3 Statistics of Ct values of reagent intra-batch imprecision test results**

| Number of days | Serial number | Medium concentration positive | | Critical concentration positive | | Negative | |
|---|---|---|---|---|---|---|---|
| | | ORF1ab | N | ORF1ab | N | ORF1ab | N |
| Day 1 | 1 | 26.30 | 25.48 | 32.03 | 32.03 | No Ct | No Ct |
| | 2 | 26.31 | 25.45 | 32.04 | 32.32 | No Ct | No Ct |
| | 3 | 26.29 | 25.51 | 31.79 | 32.37 | No Ct | No Ct |
| | 4 | 26.31 | 25.47 | 32.18 | 32.06 | No Ct | No Ct |
| | 5 | 26.31 | 25.54 | 31.88 | 32.28 | No Ct | No Ct |
| Day 2 | 6 | 26.40 | 25.58 | 31.53 | 32.42 | No Ct | No Ct |
| | 7 | 26.34 | 25.54 | 31.85 | 32.40 | No Ct | No Ct |
| | 8 | 26.36 | 25.65 | 31.73 | 32.29 | No Ct | No Ct |
| | 9 | 26.31 | 25.53 | 32.23 | 32.22 | No Ct | No Ct |
| | 10 | 26.30 | 25.54 | 32.06 | 32.14 | No Ct | No Ct |
| Day 3 | 11 | 26.37 | 25.39 | 31.88 | 32.24 | No Ct | No Ct |
| | 12 | 26.36 | 25.48 | 32.22 | 32.20 | No Ct | No Ct |
| | 13 | 26.29 | 25.47 | 32.24 | 32.30 | No Ct | No Ct |
| | 14 | 26.28 | 25.50 | 32.07 | 32.10 | No Ct | No Ct |
| | 15 | 26.33 | 25.43 | 32.27 | 32.03 | No Ct | No Ct |
| Day 4 | 16 | 25.14 | 25.40 | 31.99 | 32.19 | No Ct | No Ct |
| | 17 | 25.16 | 25.41 | 32.17 | 32.10 | No Ct | No Ct |
| | 18 | 25.15 | 25.51 | 31.76 | 32.87 | No Ct | No Ct |
| | 19 | 25.17 | 25.44 | 32.03 | 32.39 | No Ct | No Ct |
| | 20 | 25.18 | 25.41 | 32.52 | 32.91 | No Ct | No Ct |
| Day 5 | 21 | 25.16 | 25.39 | 32.10 | 32.21 | No Ct | No Ct |
| | 22 | 25.14 | 25.48 | 32.03 | 32.01 | No Ct | No Ct |
| | 23 | 25.13 | 25.45 | 31.85 | 31.92 | No Ct | No Ct |
| | 24 | 25.13 | 25.41 | 31.97 | 32.26 | No Ct | No Ct |
| | 25 | 25.17 | 25.45 | 32.06 | 32.67 | No Ct | No Ct |
| | Positive detection rate | 100% | 100% | 100% | 100% | 0% | 0% |

### Example 9. Detection of comparative example composition of the ORF1ab gene of the present invention

To further demonstrate the advantages of the specific areas mentioned in the present invention, the inventors use anther specific area on the ORF1ab gene:
ATAAAGAAATGTATCTAAAGTTGCGTAGTGATGTGCTATTACCTCTTACGCAATATA ATAGATACTTAGCTCTTTATAATAAGTACAAGTATTTTAGTGGAGCAATGGATACAA CTAGCTACAGAGAAGCTGCTTGTTGTCATCTCGCAAAGGCTCTCAATGACTTCAG TAACTCAGGTTCTGATGTTCTTTA (SEQ ID NO: 38), and on this basis, the primer and the probe are designed, and the specific sequence thereof is as shown below:
an ORF1ab forward primer 3: 5'-ATGTATCTAAAGTTGCGTAG-3' (SEQ ID NO: 39);
an ORF1ab negative primer 3: 5'-GTTACTGAAGTCATTGAGAGCCT-3' (SEQ ID NO: 40);
an ORF1ab probe 3: 5'-ATGTGCTATTACCTCTTACGCAATATAAT-3' (SEQ ID NO: 41).

First, the inventors verify the detection efficiency of primers and probes using one ORF1ab gene detection alone.

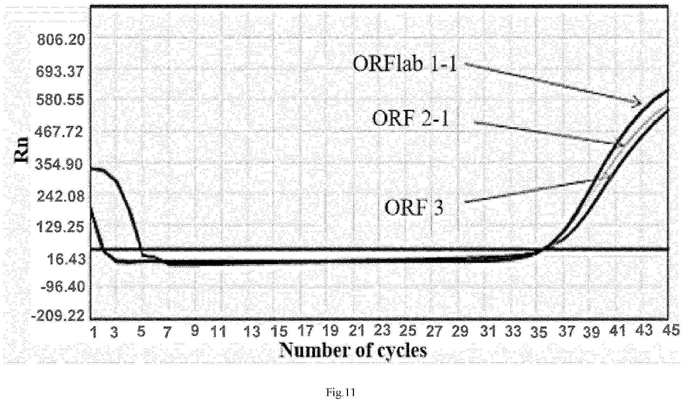

The ORF1ab gene is detected by respectively using combinations of three primers and probes: 1. an ORF1ab forward primer 1-1, an ORF1ab negative primer 1-1, and an ORF1ab probe 1-1; 2. an ORF1ab forward primer 2-1, an ORF1ab negative primer 2-1, and an ORF1ab probe 2-1; and 3. an ORF1ab forward primer 3, an ORF1ab negative primer 3, and an ORF1ab probe 3, and the results are as shown in FIG. 11. The results show that the detection efficiency of the three primers and probes on the ORF1ab gene is comparable. That is, the detection effect of a system designed for SEQ ID NO: 38 alone is comparable to that of a system designed for SEQ ID NOs: 1 and 2 alone.

SEQ ID NOs: 39-41 (the ORF1ab forward primer 3, the ORF1ab negative primer 3, and the ORF1ab probe 3) are combined with SEQ ID NOs: 8-10 mentioned in Example 1 of the present invention to form a fifth composition. SEQ ID NOs: 39-41 (the ORF1ab forward primer 3, the ORF1ab negative primer 3, and the ORF1ab probe 3) are combined with SEQ ID NOs: 14-16 mentioned in the example of the present invention to form a sixth composition.

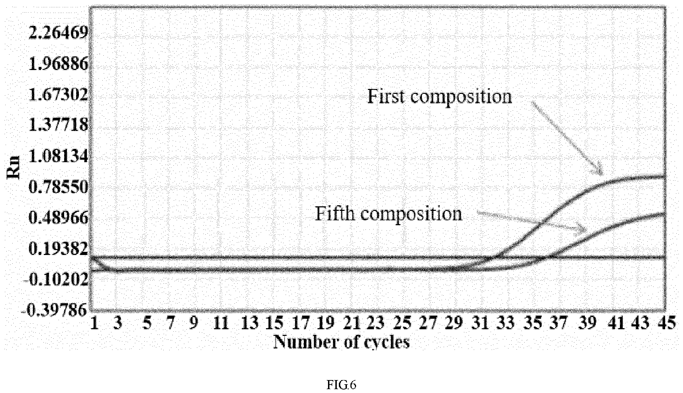

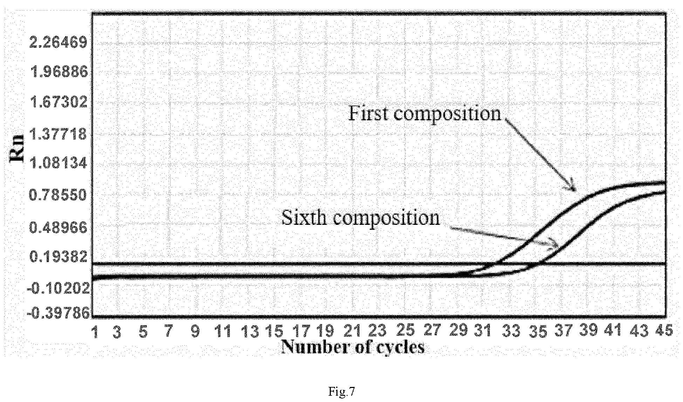

The fifth composition and the sixth composition respectively implement detection according to the method in Example 2, and the detection results of the FAM channel are as shown in FIG. 6 and FIG. 7, respectively. Although in the above-mentioned individual system verification, it is found that the detection effects of the individual primer probes are comparable (as shown in FIG. 11), after the combination, it is found that the Ct values of the fifth composition and the sixth composition are significantly moved backward than that of ORF1ab of the first composition of the present invention. Therefore, it is proved that the combination of specific areas of ORF1ab found by the present invention has the advantage of higher sensitivity in detection.

### Example 10. Detection of comparative example composition of the N gene of the present invention

To further demonstrate the advantages of the specific areas mentioned in the present invention, the inventors first design an N gene primer probe combination 2 for the above-mentioned specific area of the N gene (SEQ ID NO: 3), and the specific sequence thereof is as shown below:
an N gene forward primer 2: 5'-ACTACCTAGGAACTGGGCCAG-3' (SEQ ID NO: 43);
an N gene negative primer 2: 5'-AGCAGGATTGCGGGTGCCAAT-3' (SEQ ID NO: 44); and
an N gene probe 2: 5'-ACTTCCCTATGGTGCTAACAAAGACGGCAT-3' (SEQ ID NO: 45).

Secondly, the inventors find another specific area on the N gene:
TGAGAGCAAAATGTCTGGTAAAGGCCAACAACAACAAGGCCAAACTGTCACTAA GAAATCTGCTGCTGAGGCTTCTAAGAAGCCTCGGCAAAAACGTACTGCCACTAAAGC ATACAATGTAACACAAGCTTTCGGCAGACGTGGTCCAGAACAAACCCAAGGAAATTT TGGGGACCAGGAACTAATCAGACAAGGAACTGATTACAAACATTGGCCGCA (SEQ ID NO: 42), and on this basis, an N gene primer probe combination 3 is designed, and the specific sequence thereof is as shown below:
an N gene forward primer 3: 5'-ATGTCTGGTAAAGGCCAACAAC-3' (SEQ ID NO: 46);
an N gene negative primer 3: 5'-AGTTCCTTGTCTGATTAGTTC-3') SEQ ID NO: 47); and
an N gene probe 3: 5'-ACTGTCACTAAGAAATCTGCTGCTGAGGC-3' (SEQ ID NO: 48).

First, the inventors verify the detection efficiency of the primer probe combination using one N gene detection alone.

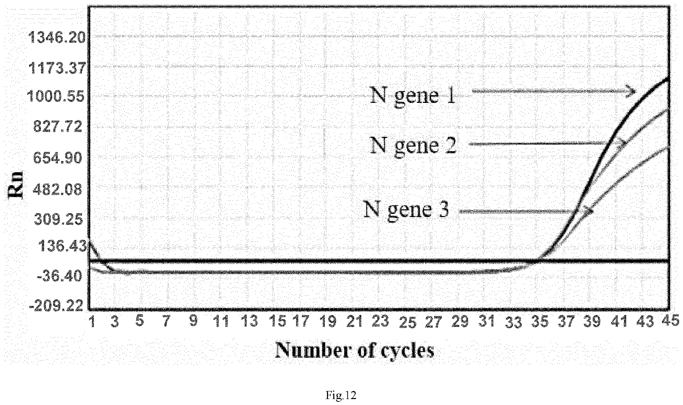

The N gene is detected by respectively using combinations of three primers and probes: 1. an N gene forward primer 1, an N gene negative primer 1, and an N gene probe 1; 2. an N gene forward primer 2, an N gene negative primer 2, and an N gene probe 2; and 3. an N gene forward primer 3, an N gene negative primer 3, and an N gene probe 3, and the results are as shown in FIG. 12. The results show that the detection efficiency of the combinations of the three primers and probes on the N gene is comparable. That is, the detection effect of a system designed for SEQ ID NO: 42 alone is comparable to that of a system designed for SEQ ID NO: 3 alone.

The N gene primer probe combination 2 is combined with SEQ ID NOs: 8-10 and SEQ ID NOs: 14-16 mentioned in Example 1 of the present invention to form a seventh composition. The N gene primer probe combination 3 is combined with SEQ ID NOs: 8-10 and SEQ ID NOs: 14-16 mentioned in the example of the present invention to form an eighth composition.

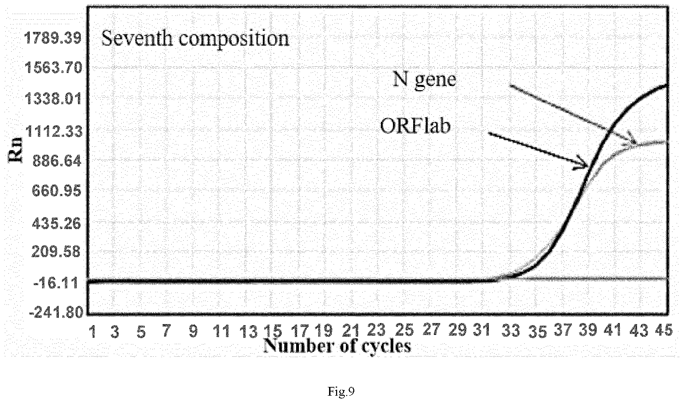

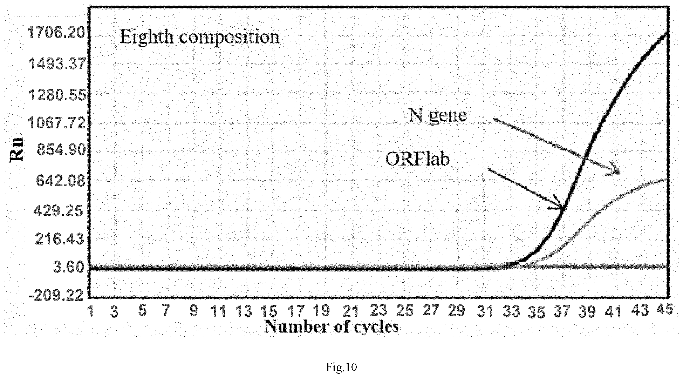

The seventh composition and the eighth composition respectively implement detection according to the method in Example 2, and the detection results of the ROX channel are as shown in FIG. 9 and FIG. 10, respectively. Although in the above-mentioned individual system verification, it is found that the detection effects of the individual primer probes are comparable (as shown in FIG. 12), after the combination, it is found that the detection efficiency of the seventh composition is comparable to that of the first composition, and the Ct value of the eighth composition is significantly moved backward than that of N gene of the first composition of the present invention. Therefore, it is proved that the specific area of the N gene found by the present invention has the advantage of higher sensitivity in combined detection.

## Claims

1. A composition for detecting SARS-CoV-2, comprising:
a first primer pair, for specifically amplifying an N gene of SARS-CoV-2:
a first probe, for detecting an amplification product of the first primer pair;
a second primer pair, for specifically amplifying an area as shown in SEQ ID NO: 1 in an ORF1ab gene;
a second probe, for detecting an amplification product of the second primer pair;
a third primer pair, for specifically amplifying an area as shown in SEQ ID NO: 2 in the ORF1ab gene; and
a third probe, for detecting an amplification product of the third primer pair.

2. The composition according to claim 1, wherein the second primer pair and the second probe are:
an ORF1ab forward primer 1-1: 5'-ATTCACCTAATTTAGCATGGCCTC-3' (SEQ ID NO: 8);
an ORF1ab negative primer 1-1: 5'-ACATCTGTCGTAGTGCAACAGGAC-3' (SEQ ID NO: 9); and
an ORF1ab probe 1-1: 5'-TTGTAACAGCTTTAAGGGCCAATTCTGCTGT-3' (SEQ ID NO: 10); or
an ORF1ab forward primer 1-2: 5'-ATGGCCTCTTATTGTAACAGCT-3' (SEQ ID NO: 11);
an ORF1ab negative primer 1-2: 5'-AGCAGTTTGTGTAGTACCGGCAGC-3' (SEQ ID NO: 12); and
an ORF1ab probe 1-2: 5'-CCAATTCTGCTGTCAAATTACAGAATA-3' (SEQ ID NO: 13).

3. The composition according to claim 1, wherein the third primer pair and the third probe are:
an ORF1ab forward primer 2-1: 5'-GAAGGTTCTGTTGCTTATGAAAGTT-3' (SEQ ID NO: 14);
an ORF1ab negative primer 2-1: 5'-TACCACTCTAACAGAACCTTC-3' (SEQ ID NO: 15); and
an ORF1ab probe 2-1: 5'-GCCCTGACACACGTTATGTGCTCATGGA-3' (SEQ ID NO: 16); or
an ORF1ab forward primer 2-2: 5'-ATGAAAGTTTACGCCCTGAC-3' (SEQ ID NO: 17);
an ORF1ab negative primer 2-2: 5'-CCGTGCCTACAGTACTCAGAATC-3' (SEQ ID NO: 18); and
an ORF1ab probe 2-2: 5'-ATGTGCTCATGGATGGCTCTATTATTCAATT-3' (SEQ ID NO: 19).

4. The composition according to any one of claims 1 to 3, wherein the first primer pair is for specifically amplifying an area as shown in SEQ ID NO: 3 in the N gene.

5. The composition according to claim 4, wherein the first primer pair and the first probe are:
an N gene forward primer 1: 5'-GGTATTTCTACTACCTAGGAACT-3' (SEQ ID NO: 5);
an N gene negative primer 1: 5'-TGCAGCATTGTTAGCAGGATTG-3' (SEQ ID NO: 6); and
an N gene probe 1: 5'-GCCAGAAGCTGGACTTCCCTATGGTGC-3' (SEQ ID NO: 7); or
an N gene forward primer 2: 5'-ACTACCTAGGAACTGGGCCAG-3' (SEQ ID NO: 43);
an N gene negative primer 2: 5'-AGCAGGATTGCGGGTGCCAAT-3' (SEQ ID NO: 44); and
an N gene probe 2: 5'-ACTTCCCTATGGTGCTAACAAAGACGGCAT-3' (SEQ ID NO: 45).

6. The composition according to claim 1, further comprising an internal standard upstream primer, an internal standard downstream primer, and an internal standard probe for monitoring, for example, the internal standard upstream primer is: 5'-GAGGTGGGACTTCAGCATGGC-3' (SEQ ID NO: 20); the internal standard downstream primer is: 5'-TGTTTCTTTTCCTTAAAGTCAAC-3' (SEQ ID NO: 21); and the internal standard probe is 5'-AGATTTGGACCTGCGAGCGGGTTCTGA-3' (SEQ ID NO: 22).

7. The composition according to claim 1, wherein in the composition, the first probe, the second probe and the third probe carry fluorescent reporter groups that do not interfere with each other.

8. Use of the composition according to any one of claims 1 to 7 in preparation of a kit for detecting SARS-CoV-2.

9. A kit for detecting SARS-CoV-2, comprising the composition according to any one of claims 1 to 7.

10. A method for detecting SARS-CoV-2, comprising the following steps:
1) extracting or releasing nucleic acids from a to-be-tested sample;
2) using the composition according to any one of claims 1 to 7 to perform fluorescent quantitative PCR on the nucleic acids obtained in step 1); and
3) obtaining and analyzing a result.

## Patentansprüche

1. Zusammensetzung zum Nachweis von SARS-CoV-2, mit:
einem ersten Primer-Paar speziell zum Verstärken eines N-Gens von SARS-CoV-2;
einer ersten Sonde zum Nachweis eines Verstärkungsprodukts für das erste Primer-Paar;
einem zweiten Primer-Paar zum speziellen Verstärken eines Bereichs, wie er in SEQ ID NR: 1 in einem ORF1ab-Gen gezeigt ist;
einer zweiten Sonde zum Nachweis eines Verstärkungsprodukts des zweiten Primer-Paars;
einem dritten Primer-Paar zum speziellen Verstärken eines Bereichs, wie er in SEQ ID NR: 2 in dem ORF1ab-Gen gezeigt ist; und
einer dritten Sonde zum Nachweis eines Verstärkungsprodukts des dritten Primer-Paars.

2. Zusammensetzung nach Anspruch 1, wobei das zweite Primer-Paar und die zweite Sonde:
ein ORF1ab-Vorwärts-Primer 1-1: 5'-ATTCACCTAATTTAGCATGGCCTC-3' (SEQ ID NR: 8);
ein ORF1ab-Negativ-Primer 1-1: 5'-ACATCTGTCGTAGTGCAACAGGAC-3' (SEQ ID NR: 9); und
eine ORF1ab-Sonde 1-1: 5'-TTGTAACAGCTTTAAGGGCCAATTCTGCTGT-3' (SEQ ID NR: 10); oder
ein ORF1ab-Vorwärts-Primer 1-2: 5'-ATGGCCTCTTATTGTAACAGCT-3' (SEQ ID NR: 11);
ein ORF1ab-Negativ-Primer 1-2: 5'-AGCAGTTTGTGTAGTACCGGCAGC-3' (SEQ ID NR: 12); und
eine ORF1ab-Sonde 1-2: 5'-CCAATTCTGCTGTCAAATTACAGAATA-3' (SEQ ID NR: 13) sind.

3. Zusammensetzung nach Anspruch 1, wobei das dritte Primer-Paar und die dritte Sonde:
ein ORF1ab-Vorwärts-Primer 2-1: 5'-GAAGGTTCTGTTGCTTATGAAAGTT-3' (SEQ ID NR: 14);
ein ORF1ab-Negativ-Primer 2-1: 5'-TACCACTCTAACAGAACCTTC-3' (SEQ ID NR: 15); und
eine ORF1ab-Sonde 2-1: 5'-GCCCTGACACACGTTATGTGCTCATGGA-3' (SEQ ID NR: 16); oder
ein ORF1ab-Vorwärts-Primer 2-2: 5'-ATGAAAGTTTACGCCCTGAC-3' (SEQ ID NR: 17);
ein ORF1ab-Negativ-Primer 2-2: 5'-CCGTGCCTACAGTACTCAGAATC-3' (SEQ ID NR: 18); und
eine ORF1ab-Sonde 2-2: 5'-ATGTGCTCATGGATGGCTCTATTATTCAATT-3' (SEQ ID NR: 19) sind.

4. Zusammensetzung nach einem der Ansprüche 1 bis 3, wobei das erste Primer-Paar zum speziellen Verstärken eines Bereichs ist, wie er in SEQ ID NR: 3 in dem N-Gen gezeigt ist.

5. Zusammensetzung nach Anspruch 4, wobei das erste Primer-Paar und die erste Sonde:
ein N-Gen-Vorwärts-Primer 1: 5'-GGTATTTCTACTACCTAGGAACT-3' (SEQ ID NR: 5);
ein N-Gen-Negativ-Primer 1: 5'-TGCAGCATTGTTAGCAGGATTG-3' (SEQ ID NR: 6); und
eine N-Gen-Sonde 1: 5'-GCCAGAAGCTGGACTTCCCTATGGTGC-3' (SEQ ID NR: 7); oder
ein N-Gen-Vorwärts-Primer 2: 5'-ACTACCTAGGAACTGGGCCAG-3' (SEQ ID NR: 43);
ein N-Gen-Negativ-Primer 2: 5'-AGCAGGATTGCGGGTGCCAAT-3' (SEQ ID NR: 44); und
eine N-Gen-Sonde 2: 5'-ACTTCCCTATGGTGCTAACAAAGACGGCAT-3' (SEQ ID NR: 45) sind.

6. Zusammensetzung nach Anspruch 1, die ferner einen vorangestellten Primer als internen Standard, einen nachgestellten Primer als internen Standard und eine Sonde als internen Standard zur Überwachung hat, wobei beispielsweise der vorangestellte Primer als interner Standard 5'-GAGGTGGGACTTCAGCATGGC-3' (SEQ ID NR: 20) ist; der nachgestellte Primer als interner Standard 5'-TGTTTCTTTTCCTTAAAGTCAAC-3' (SEQ ID NR: 21) ist; und die Sonde als interner Standard 5'-AGATTTGGACCTGCGAGCGGGTTCTGA-3' (SEQ ID NR: 22) ist.

7. Zusammensetzung nach Anspruch 1, wobei in der Zusammensetzung die erste Sonde, die zweite Sonde und die dritte Sonde fluoreszierende Meldegruppen tragen, die einander nicht stören.

8. Verwendung der Zusammensetzung nach einem der Ansprüche 1 bis 7 bei der Herstellung eines Kits zum Nachweis von SARS-CoV-2.

9. Kit zum Nachweis von SARS-CoV-2 mit der Zusammensetzung nach einem der Ansprüche 1 bis 7.

10. Verfahren zum Nachweis von SARS-CoV-2 mit den folgenden Schritten:
1) Extrahieren oder Freisetzen von Nukleinsäuren von einer zu prüfenden Probe;
2) Verwendung der Zusammensetzung nach einem der Ansprüche 1 bis 7 zur Ausführung einer fluoreszierenden quantitativen PCR an den Nukleinsäuren, die im Schritt 1) erhalten werden; und
3) Ermitteln und Auswerten eines Ergebnisses.

## Revendications

1. Composition pour la détection du SARS-CoV-2, comprenant :
une première paire d'amorces, pour amplifier spécifiquement un gène N du SARS-CoV-2 ;
une première sonde, pour détecter un produit d'amplification de la première paire d'amorces ;
une deuxième paire d'amorces, pour amplifier spécifiquement une zone telle que montrée dans la SEQ ID N° : 1 dans un gène ORF1ab ;
une deuxième sonde, pour détecter un produit d'amplification de la deuxième paire d'amorces ;
une troisième paire d'amorces, pour amplifier spécifiquement une zone telle que montrée dans la SEQ ID N° : 2 dans le gène ORF1ab ; et
une troisième sonde, pour détecter un produit d'amplification de la troisième paire d'amorces.

2. Composition selon la revendication 1, dans laquelle la deuxième paire d'amorces et la deuxième sonde sont :
une amorce directe ORFlab, 1-1 : 5'-ATTCACCTAATTTAGCATGGCCTC-3' (SEQ ID N° : 8) ;
une amorce négative ORFlab, 1-1 : 5'-ACATCTGTCGTAGTGCAACAGGAC-3' (SEQ ID N° : 9) ; et
une sonde ORFlab, 1-1 : 5'-TTGTAACAGCTTTAAGGGCCAATTCTGCTGT-3' (SEQ ID N° : 10) ; ou
une amorce directe ORFlab, 1-2 : 5'-ATGGCCTCTTATTGTAACAGCT-3' (SEQ ID N° : 11) ;
une amorce négative ORFlab, 1-2 : 5'-AGCAGTTTGTGTAGTACCGGCAGC-3' (SEQ ID N° : 12) ; et
une sonde ORFlab, 1-2 : 5'-CCAATTCTGCTGTCAAATTACAGAATA-3' (SEQ ID N° : 13).

3. Composition selon la revendication 1, dans laquelle la troisième paire d'amorces et la troisième sonde sont :
une amorce directe ORFlab, 2-1 : 5'-GAAGGTTCTGTTGCTTATGAAAGTT-3' (SEQ ID N° : 14) ;
une amorce négative ORFlab, 2-1 : 5'-TACCACTCTAACAGAACCTTC-3' (SEQ ID N° : 15) ; et
une sonde ORFlab, 2-1 : 5'-GCCCTGACACACGTTATGTGCTCATGGA-3' (SEQ ID N° : 16) ; ou
une amorce directe ORFlab, 2-2 : 5'-ATGAAAGTTTACGCCCTGAC-3' (SEQ ID N° : 17) ;
une amorce négative ORFlab, 2-2 : 5'-CCGTGCCTACAGTACTCAGAATC-3' (SEQ ID N° : 18) ; et
une sonde ORFlab, 2-2 : 5'-ATGTGCTCATGGATGGCTCTATTATTCAATT-3' (SEQ ID N° : 19).

4. Composition selon l'une quelconque des revendications 1 à 3, dans laquelle la première paire d'amorces est destinée à amplifier spécifiquement une zone telle que représentée dans la SEQ ID N° : 3 dans le gène N.

5. Composition selon la revendication 4, dans laquelle la première paire d'amorces et la première sonde sont :
une amorce directe du gène N, 1 : 5'-GGTATTTCTACTACCTAGGAACT-3' (SEQ ID N° : 5) ;
une amorce négative du gène N, 1 : 5'-TGCAGCATTGTTAGCAGGATTG-3' (SEQ ID N° : 6) ; et
une sonde du gène N, 1 : 5'-GCCAGAAGCTGGACTTCCCTATGGTGC-3' (SEQ ID N° : 7) ; ou
une amorce directe du gène N, 2 : 5'-ACTACCTAGGAACTGGGCCAG-3' (SEQ ID N° : 43) ;
une amorce négative du gène N, 2 : 5'-AGCAGGATTGCGGGTGCCAAT-3' (SEQ ID N° : 44) ; et
une sonde du gène N, 2 : 5'-ACTTCCCTATGGTGCTAACAAAGACGGCAT-3' (SEQ ID N° : 45).

6. Composition selon la revendication 1, comprenant en outre une amorce amont standard interne, une amorce aval standard interne, et une sonde standard interne de surveillance, où par exemple l'amorce amont standard interne est : 5'-GAGGTGGGACTTCAGCATGGC-3' (SEQ ID N° : 20) ; l'amorce aval standard interne est : 5'-TGTTTCTTTTCCTTAAAGTCAAC-3' (SEQ ID N° : 21) ; et la sonde standard interne est 5'-AGATTTGGACCTGCGAGCGGGTTCTGA-3' (SEQ ID N° : 22).

7. Composition selon la revendication 1, dans laquelle, dans la composition, la première sonde, la deuxième sonde et la troisième sonde portent des groupes rapporteurs fluorescents qui n'interfèrent pas les uns avec les autres.

8. Utilisation de la composition selon l'une quelconque des revendications 1 à 7 dans la préparation d'un kit de détection du SARS-CoV-2.

9. Kit de détection du SARS-CoV-2, comprenant la composition selon l'une quelconque des revendications 1 à 7.

10. Procédé de détection du SARS-CoV-2, comprenant les étapes suivantes :
1) extraction ou libération d'acides nucléiques d'un échantillon à tester ;
2) utilisation de la composition selon l'une quelconque des revendications 1 à 7 pour mettre en œuvre une PCR quantitative fluorescente sur les acides nucléiques obtenus à l'étape 1) ; et
3) obtention et analyse d'un résultat.
